# EUROPEAN PATENT APPLICATION

(11) **EP 1 905 755 A1**
(43) Date of publication of application: **02.04.2008**
(21) Application number: 06020111.8
(22) Date of filing: 26.09.2006
(51) Int. Cl.: C07C 17/269, C07C 25/18

(54) **Process for the synthesis of 2,2 ,6-tribromobiphenyl**

(71) Applicant: Lonza AG, 4052 Basel (CH)
(72) Inventor: The designation of the inventor has not yet been filed

(57) **Abstract**

A process for the synthesis of 2,2',6-tribromobiphenyl

## Description

The invention is directed to a process for the synthesis of 2,2',6-tribromobiphenyl.

2,2',6-Tribromobiphenyl (TBBP) has turned out to be a suitable starting material for the synthesis of several biphenyl compounds. Such compounds are valuable precursors for example in the materials sciences and the preparation of ligands for the use in the synthesis of chiral compounds for the pharmaceutical industry. A synthesis of this compound is known: the first step consists of an Ullmann coupling of 1,3-dibromo-2-iodobenzene which affords 2,2',6,6'-tetrabromobiphenyl. Next, debromination by halogen/metal exchange and hydrolysis gives 2,2',6-tribromobiphenyl in an overall yield of 30 % according to WO-A-2006/006065. On the other hand, the "aryne coupling" between 1,3-dibromo-2-iodobenzene and 1,2-dibromobenzene introduced by Leroux et al., Angew. Chem. 2002, 41, 4272-4274 affords 2,2',6-tribromobiphenyl in a poor yield of 40%. Furthermore, the latter synthesis is suitable for very small reaction sizes only. Until today the synthesis of TBBP is carried out using the two step process using the Ullmann coupling and debromination as outlined above due to the limitation of the "aryne coupling" between 1,3-dibromo-2-iodobenzene and 1,2-dibromobenzene. Because of an increasing demand of biphenyl ligands the demand of TBBP increased too. A main task for the present invention was therefore to supply an alternative process with remarkably increased yields.

The problem is solved by the process of claim 1.

Claimed is a process for the preparation of 2,2',6-tribromobiphenyl of formula 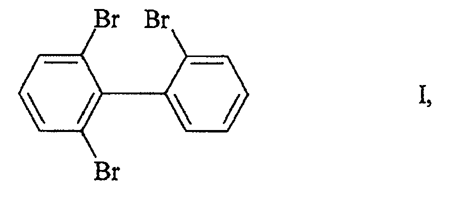 said process comprising the steps of
a) mixing an organometallic compound with 1,3-dibromo-2-iodobenzene at a molar ratio of at least 1:1, and
b) adding 1,2-dibromobenzene to the mixture in a molar ratio, based on 1,3-dibromo-2-iodobenzene, of 0.9:1 to 1.1:1,
to obtain 2,2',6-tribromobiphenyl which is subsequently subjected to standard work-up procedures.

Preferably the ratio of the organometallic compound to 1,3-dibromo-2-iodobenzene is in the range of 1:1 to 3:1. More preferably the range is 1.5:1 to 2.5:1, most preferably it is 2:1.

In a preferred embodiment the organometallic compound is added very slowly to 1,3-di-bromo-2-iodobenzene. A suitable addition time depends on the dilution and molar amount of the organometallic compound to be added.

An organometallic compound suitable for the present process must be able to undergo a halogen-metal exchange with 1,3-dibromo-2-iodobenzene forming a compound of the formula 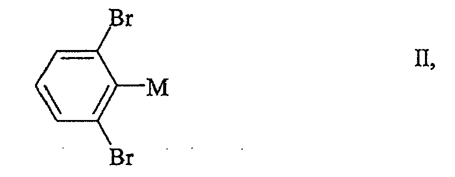 wherein M represents a monovalent metal atom like lithium (Li) or in case of a polyvalent metal like magnesium (Mg) the metal atom including any substituents which are not involved in the halogen-metal exchange. Suitable organometallic compounds comprise a metal atom and at least one organic moiety having a carbon-metal bond. Optionally each organic moiety comprises one or more halogen atoms. By reacting the organometallic compound with 1,3-dibromo-2-iodobenzene the iodine atom is exchanged with the metal atom while one organic moiety is removed from the organometallic compound. In the case of polyvalent metals or metal complexes comprising at least one polyvalent metal, all further metal, inorganic (e.g halogen atoms) or organic moieties (e.g. alkyl, ferrocenyl, or phthalocyanine) remain bound to the metal atom or metal complex.

In a preferred embodiment the organometallic compound is a Grignard reagent or a lithiation reagent.

Examples for suitable Grignard reagents are ethylmagnesium chloride, ethylmagnesium bromide, isopropylmagnesium chloride, isopropylmagnesium bromide and "magnesate" complexes. Magnesate complexes are for example lithium tri-*n*-butylmagnesate, [2-(*N,N*-dimethylaminomethyl)ferrocenyl]magnesium compounds like Li₂Mg(FcN)₂Br₂•(OEt₂)₂, wherein Fc is ferrocenyl or halophthalocyaninato magnesate complex salts like [Mg(X)Pc²⁻]•(*n*-Bu₄N) or [Mg(X)Pc²⁻]•(PNP), wherein Pc is phthalo-cyanin and X is F, Cl or Br. Said magnesate complex salts can be obtained for example by reacting magnesium phthalocyanine with tetra(*n*-butyl)ammonium halide ((*n*-Bu₄N)X, wherein X is F, Cl or Br) or with bis(triphenylphosphine)iminium halide ((PNP)X, wherein X is F, Cl or Br).

In a preferred embodiment the organometallic compound is a lithiation reagent, preferably is *n*-butyllithium, *tert*-butyllithium or phenyllithium, more preferably is *tert*-butyllithium.

By using Grignard reagents for the reaction the temperature control is not very important and the reaction can be carried out at temperatures up to room temperature. Using lithiation reagents and especially *n*-butyllithium, *tert*-butyllithium or phenyllithium a good temperature control throughout the whole step a) is important.

In a preferred embodiment using lithiation reagents the temperature in step a) is maintained at or below -75 °C, more preferably at or below -90 °C and even more preferably at or below -100 °C.

In a preferred process regarding all organometallic compounds reaction step a) is carried out in a non-polar solvent. Preferably the solvent is selected from the group consisting of tetrahydrofuran, 1,4-dioxane, cyclohexane, diethyl ether, *tert*-butyl methyl ether, diisopropyl ether, benzene, toluene, and mixtures thereof.

In a further preferred process reaction step b) is carried out in a non-polar solvent. Preferably the solvent is selected from the group consisting of tetrahydrofuran, 1,4-dioxane, cyclohexane, diethyl ether, *tert*-butyl methyl ether, diisopropyl ether, benzene, toluene, and mixtures thereof

Preferably both steps are carried out in the same solvent.

Polar or protic solvents like chloroform, dichloromethane, all kind of alcohols and acetonitrile are not suitable to perform the reaction.

### Examples:

### Example 1:

### 1,3-Dibromo-2-iodobenzene

1,3-Dibromobenzene (47.2 g, 24.2 mL, 200 mmol, 1 eq) was added dropwise to a solution of lithium diisopropylamide (LDA, 200 mmol, 1 eq) in tetrahydrofuran (400 mL) at -78 °C. After 2 h, a solution of iodine (50.8 g, 200 mmol, 1 eq) in tetrahydrofuran (200 mL) was added and the reaction mixture was then allowed to reach 25 °C. The solvent was evaporated and the residue was taken up in diethyl ether. The organic layer was washed with a saturated solution of sodium sulfite (3×100 mL), dried and evaporated. Crystallization from ethanol afforded 1,3-dibromo-2-iodobenzene (60.6 g, 167 mmol, 84%) as white platelets; m.p. 99 - 100 °C. - ¹H NMR (300 MHz, CDCl₃) : δ = 7.47 (2 H, d, *J* = 8 Hz), 6.99 (1 H, t, *J* = 7.9 Hz). - ¹³C NMR (75 MHz, CDCl₃): δ = 131.3, 131.0, 130.3, 109.4.- C₆H₃Br₂I (361.8): calculated C 19.92%, H 0.84%; found C 19.97%, H 0.80%.

### Example 2:

### 2,2',6-Tribromobiphenyl

To a solution of 1,3-dibromo-2-iodobenzene of example 1 (13.7 g, 37.9 mmol, 1 eq) in tetrahydrofuran (160 mL) at -100 °C *tert*-butyllithium (75.9 mmol, 2 eq) in pentane (44.6 mL) was slowly added in the course of 3 h. After additional 1 h, 1,2-dibromobenzene (8.94 g, 4.57 mL, 37.9 mmol) was added dropwise and the reaction mixture was then allowed to reach 25 °C. After 15 h, distilled water (150 mL) was added, followed by extraction with ethyl acetate (3×150 mL). The combined organic layers were dried, filtered and evaporated. The residue was purified by flash chromatography which afforded 2,2',6-tribromobiphenyl (12.9 g, 33 mmol, 97%) as white needles; m.p. 95 -97 °C.- ¹H NMR (400 MHz, CDCl₃) : δ = 7.69 (1 H, d, *J*= 8.1 Hz), 7.64 (2 H, dd, *J*= 8.1, 0.7 Hz), 7.42 (1 H, tt, *J=* 7.5, 0.9 Hz), 7.29 (1 H, ddt, *J=* 7.8, 1.8, 0.7 Hz), 7.18 (1 H, dd, *J=* 7.6, 1.6 Hz), 7.12 (1 H, dd, *J*= 8.1, 0.7 Hz).- ¹³C NMR (101 MHz, CDCl₃): δ = 142.2, 141.9, 132.6, 131.4, 130.6, 130.3, 129.8, 127.4, 124.4, 123.3. - C₁₂H₇Br₃ (390.7): calculated C 36.87%, H 1.81%; found C 36.82%, H 1.66%.

### Comparative Example 1:

### 2,2',6-Tribromobiphenyl

Butyllithium (25 mmol, 1 eq) in hexanes (15 mL) was added dropwise to a solution of 1,3-dibromo-2-iodobenzene of example 1 (9.0 g, 25 mmol, 1 eq) in tetrahydrofuran (50 mL) at -100 °C. After 45 min, 1,2-dibromobenzene (5.9 g, 3.0 mL, 37.9 mmol) was added dropwise and the reaction mixture was then allowed to reach 25 °C. After 15 h, distilled water (100 mL) was added, followed by extraction with ethyl acetate (3×100 mL). The combined organic layers were dried, filtered and evaporated. The residue was purified by flash chromatography which afforded 2,2',6-tribromobiphenyl (4.3 g, 11 mmol, 43%) as white needles; m.p. 95 -97 °C.- ¹H NMR (400 MHz, CDCl₃) corresponds.

### Comparative Example 2:

### 2,2',6,6'-Tetrabromobiphenyl

Butyllithium (14 mmol) in hexanes (5.6 mL) was added to a solution of 1,3-dibromo-2-iodobenzene (4.3 g, 12 mmol) in diethyl ether (180 mL) at -75 °C. After the solution was stirred for additional 2 h at -75 °C, copper(II)chloride (9.7 g, 72 mmol) was added, and the reaction mixture was allowed to attain 25 °C over a 12 h period. Cold water was added to the reaction mixture and the organic layer was separated. The aqueous phase was extracted with ethyl acetate (2 × 100 mL). The combined organic layers were dried over sodium sulfate before being evaporated. 2,2',6,6'-Tetrabromobiphenyl precipitated upon treatment of the residue with hexanes cooled to -20 °C. The product (9.0 g, 33%) was pure enough for further reaction in Comparative Example 3; m.p. 214 to 215 °C; ¹H NMR (CDCl₃, 400 MHz): δ = 7.67 (d, *J* = 8.3 Hz, 4 H), 7.17 (t, *J*= 8.0 Hz, 2 H).

### Comparative Example 3:

### 2,2',6-Tribromobiphenyl

At -75 °C, butyllithium (100 mmol) in hexanes (52 mL) was added to a solution of 2,2',6,6'-tetrabromobiphenyl (47 g, 100 mmol) in tetrahydrofuran (500 mL). Immediately after the addition was completed, methanol (10 mL) was added and, after addition of water (200 mL), the organic phase was separated and the aqueous layer was extracted with diethyl ether (2×100 mL). The combined organic layers were dried over sodium sulfate before being evaporated. Crystallization from ethanol (500 mL) afforded 35 g (91%) 2,2',6-tribromobiphenyl as colorless needles; m.p. 95 to 97 °C; ¹H-NMR (CDCl₃, 400 MHz) corresponds.

The comparative examples 2 and 3 show a total yield of about 30% based on 1,3-dibromo-2-iodobenzene.

## Claims

1. A process for the preparation of 2,2',6-tribromobiphenyl of formula 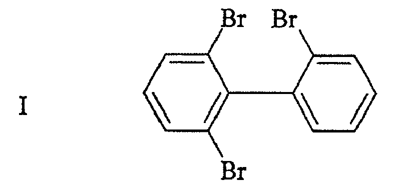 said process comprising the steps of
a) mixing an organometallic compound with 1,3-dibromo-2-iodobenzene in a molar ratio of at least 1:1, and
b) adding 1,2-dibromobenzene to the mixture in a molar ratio, based on 1,3-dibromo-2-iodobenzene, of 0.9:1 to 1.1:1,
to obtain 2,2',6-tribromobiphenyl.

2. The process of claim 1, wherein the ratio of the organometallic compound to 1,3-di-bromo-2-iodobenzene is in the range of 1:1 to 3:1.

3. The process of claims 1 or 2, wherein the organometallic compound is a Grignard reagent or a lithiation reagent.

4. The process of claim 3, wherein the Grignard reagent is selected from the group consisting of ethylmagnesium chloride, ethylmagnesium bromide, isopropylmagnesium chloride, isopropylmagnesium bromide and magnesate complexes.

5. The process of claim 3, wherein the lithiation reagent is selected from the group consisting of *n*-butyllithium, *sec*-butyllithium, *tert*-butyllithium and phenyllithium.

6. The process of claim 5, wherein the lithiation reagent is *tert*-butyllithium.

7. The process of claims 5 or 6, wherein the temperature in step a) is maintained at or below -75 °C, preferably at or below -90 °C and more preferably at or below -100°C.

8. The process of any of claims 1 to 7, wherein the reaction step a) is carried out in a non-polar solvent, preferably selected from the group consisting of tetrahydrofuran, 1,4-dioxane, cyclohexane, diethyl ether, *tert*-butyl methyl ether, diisopropyl ether, benzene and toluene.

9. The process of any of claims 1 to 8, wherein the reaction step b) is carried out in a non-polar solvent, preferably selected from the group consisting of tetrahydrofuran, 1,4-dioxane, cyclohexane, diethyl ether, *tert*-butyl methyl ether, diisopropyl ether, benzene and toluene.
